# EUROPEAN PATENT SPECIFICATION

(11) **EP 4 060 037 B1**
(45) Date of publication and mention of the grant of the patent: **21.08.2024**
(21) Application number: 21739223.2
(22) Date of filing: 19.04.2021
(51) Int. Cl.: C12N 9/90, C12N 15/70, C12P 13/22

(54) **TAUTOMERASE PPTA VARIANT, AND METHOD FOR PRODUCING L-TRYPTOPHAN USING SAME**
TAUTOMERASE-PPTA-VARIANTE UND VERFAHREN ZUR HERSTELLUNG VON L-TRYPTOPHAN DAMIT
VARIANT DE TAUTOMÉRASE PPTA, ET PROCÉDÉ DE PRODUCTION DE L-TRYPTOPHANE À L'AIDE DE CELUI-CI

(30) Priority: 25.01.2021 KR 20210010278
(43) Date of publication of application: 21.09.2022
(73) Proprietor: CJ Cheiljedang Corporation, Seoul 04560 (KR)
(72) Inventor: PARK, Goun, Seoul 04560 (KR); CHANG, Jin Sook, Seoul 04560 (KR); JUNG, Moo Young, Seoul 04560 (KR); KIM, Hyun Ah, Seoul 04560 (KR); BAE, Jee Yeon, Seoul 04560 (KR); KIM, Hyo Jin, Seoul 04560 (KR); SEO, Chang Il, Seoul 04560 (KR)
(74) Representative: Mewburn Ellis LLP
(86) International application number: PCT/KR2021/004871
(87) International publication number: WO 2022/158647

(56) References cited:
- EP-A1- 3 599 246
- WO-A1-2020/204427
- KR-A- 20200 118 334
- KR-B1- 100 838 037
- KR-B1- 101 991 206
- KR-B1- 101 996 767
- US-A1- 2018 127 792
- DATABASE Protein [online] 1 June 2019 (2019-06-01), ANONYMOUS: "tautomerase PptA [Escherichia coli]", XP055829873, retrieved from NCBI accession no. WP_096198730 Database accession no. WP_096198730
- DATABASE Protein 1 June 2019 (2019-06-01), ANONYMOUS: "tautomerase PptA [Escherichia coli]", XP055829873, retrieved from NCBI Database accession no. WP_096198730

## Description

### [Technical Field]

The present invention relates to a novel tautomerase pptA variant, an *Escherichia coli* strain comprising the variant, and a method for producing L-tryptophan using the strain.

### [Background Art]

Various studies are being conducted to develop highly efficient microorganisms and fermentation process technologies for the production of L-amino acids, and other useful substances. For example, a target substance-specific approach is mainly used in which the expression of a gene encoding an enzyme involved in L-tryptophan biosynthesis is increased, or in which genes unnecessary for the biosynthesis are removed (US 8945907 B2). KR100838037 discloses a manufacturing method of L-tryptophan using a recombinant microorganism an increased production efficiency of L-tryptophan wherein the *entC* gene coding isochorismate synthase of from *Escherichia coli* is inactivated. WO2020204427 discloses a microorganism with L-tryptophan-releasing protein or exporter and, a method of producing L-tryptophan using the same. US2018127792 discloses variants of *E*. *coli* for the production of L-tryptophan wherein the activity of a phosphatase is inactivated. The sequence of the wild type tautomerase PptA from *Escherichia coli* was provided in the GenPept database from NCBI with accession number WP_096198730.1.

However, it is still necessary to conduct studies to effectively increase the L-tryptophan producing ability as the demand for L-tryptophan increases.

### [Disclosure]

### [Technical Problem]

The present inventors have developed a novel tautomerase pptA variant for increasing L-tryptophan production, an *Escherichia coli* strain comprising the variant, and a method for producing L-tryptophan using the strain, thereby completing the present disclosure.

### [Technical Solution]

An object of the present invention is to provide a tautomerase pptA variant consisting of an amino acid sequence represented by SEQ ID NO: 1, in which arginine, which is an amino acid corresponding to position 31 of an amino acid sequence of SEQ ID NO: 3, is substituted with histidine. The invention is limited to the subject matter as defined in the claims.

Another object of the present invention is to provide a polynucleotide encoding the variant of the present invention.

Still another object of the present invention is to provide an *Escherichia coli* strain that comprises the variant of the present invention or a polynucleotide encoding the variant and has an L-tryptophan producing ability.

Still another object of the present invention is to provide a method for producing L-tryptophan, which comprises culturing an *Escherichia coli* strain that comprises the variant of the present invention or a polynucleotide encoding the variant and has an L-tryptophan producing ability in a medium.

### [Advantageous Effects]

In the case of culturing an *Escherichia coli* strain comprising the tautomerase pptA variant of the present invention, it is possible to produce L-tryptophan at a higher yield as compared to the case of existing microorganisms having unmodified polypeptides.

### [Best Mode]

The present invention will be described in detail as follows. Throughout the present specification, a number of papers and patent documents are referenced and citations thereof indicated.

The scope of the invention is defined by the claims.

An aspect of the present invention provides a variant consisting of an amino acid sequence represented by SEQ ID NO: 1, in which arginine, which is an amino acid corresponding to position 31 of an amino acid sequence of SEQ ID NO: 3, is substituted with histidine.

The variant of the present disclosure may have, comprise, or consist essentially of the amino acid sequence represented by SEQ ID NO: 1.

In the variant of the present disclosure, the amino acid corresponding to position 31 based on the amino acid sequence of SEQ ID NO: 3 in the amino acid sequence represented by SEQ ID NO: 1 is histidine.

The "conservative substitution" means the substitution of one amino acid with another amino acid having similar structural and/or chemical properties. Such an amino acid substitution may generally occur based on similarity in the polarity, charge, solubility, hydrophobicity, hydrophilicity, and/or amphipathic nature of the residues. Usually, conservative substitution may hardly affect or not affect the activity of the proteins or polypeptides.

For the purposes of the present disclosure, the variant may be a polypeptide comprising an amino acid sequence represented by SEQ ID NO: 1, in which arginine, which is an amino acid corresponding to position 31 of the amino acid sequence of SEQ ID NO: 3, is substituted with histidine.

A signal (or leader) sequence that is co-translationally or post-translationally involved in the protein translocation may be conjugated to the N-terminus of the variant. The variant may be conjugated with other sequences or linkers so as to be identified, purified, or synthesized.

In the present disclosure, the term "homology" or "identity" means the degree of similarity between two given amino acid sequences or base sequences and may be expressed as a percentage. The terms homology and identity may often be used interchangeably.

The sequence homology or identity of a conserved polynucleotide or polypeptide is determined by standard alignment algorithms, and the default gap penalty established by a program used may be used together. Substantially, homologous or identical sequences are generally capable of being hybridized with the entirety or a part of the sequence in moderately or highly stringent conditions. It is apparent that hybridization also includes hybridization of a polynucleotide with a polynucleotide comprising a general codon or a codon in consideration of codon degeneracy.

Whether any two polynucleotide or polypeptide sequences have homology, similarity, or identity may be determined using known computer algorithms such as the "FASTA" program, for example, using default parameters as in Pearson et al., (1988) Proc. Natl. Acad. Sci. USA 85:2444. Alternatively, the homology, similarity, or identity may be determined using Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (including GCG program package (Devereux, J., et al., Nucleic Acids Research 12:387 (1984)), BLASTP, BLASTN, FASTA (Atschul, S. F., et al., J MOLEC BIOL 215:403 (1990); Guide to Huge Computers, Martin J. Bishop, ed., Academic Press, San Diego, 1994; and CARILLO et al. (1988) SIAM J Applied Math 48:1073). For example, BLAST of the National Center for Biotechnology Information or ClustalW may be used to determine the homology, similarity, or identity.

The homology, similarity, or identity of polynucleotides or polypeptides may be determined by comparing sequence information using, for example, a GAP computer program such as Needleman et al. (1970), J Mol Biol. 48:443 as announced in, for example, Smith and Waterman, Adv. Appl. Math (1981) 2:482. In summary, the GAP program may be defined as the value acquired by dividing the number of similarly aligned symbols (namely, nucleotides or amino acids) by the total number of symbols in the shorter of two sequences. The default parameters for the GAP program may include (1) a binary comparison matrix (including values of 1 for identity and 0 for non-identity) and a weighted comparison matrix of Gribskov et al., (1986) Nucl. Acids Res. 14:6745 (or EDNAFULL (EMBOSS version of NCBI NUC4.4) substitution matrix) as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

As an example of the present invention, the variant of the present invention exhibits tautomerase pptA activity. Additionally, the variant of the present invention may exhibit activity so as to have an increased L-tryptophan producing ability as compared to a wild-type polypeptide exhibiting tautomerase pptA activity.

As used herein, the term "tautomerase pptA" refers to a tautomerase (isomerase), which uses an enol isomer of phenylpyruvate, 2-hydroxy-2,4-pentadienoate, or (*p*-hydroxyphenyl)pyruvate as a substrate. Specifically, the "tautomerase pptA" of the present invention may be used interchangeably with "4-oxalocrotonate tautomerase" or "4-OT". The sequence of the tautomerase pptA can be obtained from GenBank of the NCBI, a known database. Specifically, the tautomerase pptA may be a polypeptide having tautomerase pptA activity encoded by the *pptA* gene, but is not limited thereto.

In the present disclosure, the term "corresponding to" refers to amino acid residues at positions listed in the polypeptide or amino acid residues that are similar, identical, or homologous to those listed in the polypeptide. Identifying the amino acid at the corresponding position may be determining a specific amino acid in a sequence that refers to a specific sequence. As used herein, "corresponding region" generally refers to a similar or corresponding position in a related protein or a reference protein.

For example, an arbitrary amino acid sequence is aligned with SEQ ID NO: 3, and based on this, each amino acid residue of the amino acid sequence may be numbered with reference to the amino acid residue of SEQ ID NO: 3 and the numerical position of the corresponding amino acid residue. For example, a sequence alignment algorithm as described in the present disclosure may determine the position of an amino acid or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48:443-453), the Needleman program of the EMBOSS package (EMBOSS: The European Molecular Biology Open Software Suite, Rice et al., 2000, Trends Genet. 16:276-277), and the like may be used, but the program and algorithm are not limited thereto, and a sequence alignment program, a pairwise sequence comparison algorithm, and the like known in the art may be appropriately used.

Another aspect of the present invention is to provide a polynucleotide encoding the variant of the present invention.

In the present disclosure, the term "polynucleotide" is a DNA or RNA strand having a certain length or more as a polymer of nucleotides in which nucleotide monomers are connected in a long chain by covalent bonds, and more specifically means a polynucleotide fragment encoding the variant.

The polynucleotide encoding the variant of the present invention comprises a base sequence encoding the amino acid sequence represented by SEQ ID NO: 1. As an example of the present invention, the polynucleotide of the present invention may have or comprise the sequence of SEQ ID NO: 2. The polynucleotide of the present invention may consist of or consist essentially of the sequence of SEQ ID NO: 2.

In the polynucleotide of the present invention, various modifications may be made in the coding region as long as the amino acid sequence of the variant of the present invention is not changed in consideration of codon degeneracy or codons preferred in organisms that are intended to express the variant of the present invention. Specifically, the polynucleotide of the present invention has or comprises a base sequence having 70% or more, 75% or more, 80% or more, 85% or more, 90% or more, 95% or more, 96% or more, 97% or more, 98% or more, but less than 100% homology or identity to the sequence of SEQ ID NO: 2 or may consist of or consist essentially of a base sequence having 70% or more, 75% or more, 80% or more, 85% or more, 90% or more, 95% or more, 96% or more, 97% or more, 98% or more, but less than 100% homology or identity to the sequence of SEQ ID NO: 2, but is not limited thereto. Here, in the sequence having homology or identity, the codon encoding the amino acid corresponding to position 31 of SEQ ID NO: 1 may be one of the codons encoding histidine.

The polynucleotide of the present invention may comprise a probe that can be prepared from a known gene sequence, for example, a sequence without limitation as long as it is a sequence that can hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof include a condition in which polynucleotides having higher homology or identity, namely polynucleotides having 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed one time, specifically two to three times at a salt concentration and temperature equivalent to 60°C, 1X SSC, 0.1% SDS, specifically 60°C, 0.1X SSC, 0.1% SDS, more specifically 68°C, 0.1X SSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

Specifically, a polynucleotide having homology or identity to the polynucleotide of the present invention may be detected using a hybridization condition including a hybridization step at a Tₘ value of 55°C and the above-described conditions. The Tₘ value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (for example, J. Sambrook et al., Molecular Cloning, A Laboratory Manual, 2nd Edition, Cold Spring Harbor Laboratory press, Cold Spring Harbor, New York, 1989).

Another aspect of the present disclosure is to provide a vector comprising the polynucleotide of the present disclosure. The vector may be an expression vector for expressing the polynucleotide in a host cell, but is not limited thereto.

The vector of the present disclosure may include a DNA construct comprising a polynucleotide sequence encoding a polypeptide of interest operably linked to a suitable expression regulatory region (or expression control sequence) so that the polypeptide of interest may be expressed in a suitable host. The expression regulatory region may contain a promoter capable of initiating transcription, any operator sequence for controlling the transcription, a sequence encoding a suitable mRNA ribosome binding site, and a sequence controlling termination of transcription and translation. The vector may be transformed into a suitable host cell and then replicated or function independently of the host genome, or may be integrated into the genome itself.

The vector used in the present disclosure is not particularly limited, but any vector known in the art may be used. Examples of commonly used vectors include natural or recombinant plasmids, cosmids, viruses, and bacteriophages. For example, pWE15, M13, MBL3, MBL4, IXII, ASHII, APII, t10, t11, Charon4A, Charon21A, and the like may be used as a phage vector or a cosmid vector, and pDZ system, pBR system, pUC system, pBluescript II system, pGEM system, pTZ system, pCL system, pET system, and the like may be used as a plasmid vector. Specifically, pDZ, pDC, pDCM2, pACYC177, pACYC184, pCL, pECCG117, pUC19, pBR322, pMW118, and pCC1BAC vectors and the like may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further contain a selection marker for the confirmation of chromosome insertion. The selection marker is for selecting the cells transformed with vectors, that is, for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells can be selected.

In the present disclosure, the term "transformation" means that a vector comprising a polynucleotide encoding a target polypeptide is introduced into a host cell or a microorganism so that the polypeptide encoded by the polynucleotide can be expressed in the host cell. The transformed polynucleotide may be located by being inserted into the chromosome of the host cell or located outside the chromosome as long as it can be expressed in the host cell. The polynucleotide comprises DNA and/or RNA encoding a polypeptide of interest. The polynucleotide may be introduced in any form as long as it can be introduced into a host cell and expressed. For example, the polynucleotide may be introduced into a host cell in the form of an expression cassette, which is a gene construct containing all elements required for self-expression. The expression cassette may usually contain a promoter operably linked to the polynucleotide, a transcription termination signal, a ribosome binding site, and a translation termination signal. The expression cassette may be in the form of an expression vector capable of self-replicating. The polynucleotide may be introduced into a host cell in its own form and operably linked to a sequence required for expression in the host cell, but is not limited thereto.

In the above, the term "operably linked" means that the polynucleotide sequence is functionally linked to a promoter sequence that initiates and mediates transcription of the polynucleotide encoding the variant of interest of the present disclosure.

Still another aspect of the present invention is to provide an *Escherichia coli* strain that comprises the variant of the present invention or the polynucleotide of the present invention.

The strain of the present invention may comprise a modified polypeptide of the present invention, a polynucleotide encoding the polypeptide, or a vector comprising the polynucleotide of the present disclosure.

In the present invention, the "strain (or microorganism)" includes all wild-type microorganisms or naturally or artificially genetically modified microorganisms, and it may be a microorganism in which a specific mechanism is weakened or strengthened due to a insertion of an external gene or an activity enhancement or inactivation of an endogenous gene, and it may be a microorganism comprising genetic modification for production of a polypeptide, protein, or product of interest.

The strain of the present invention may be a strain comprising any one or more of the variant of the present invention, the polynucleotide of the present invention, or a vector comprising the polynucleotide of the present invention; a strain modified to express the variant of the present invention or the polynucleotide of the present invention; a strain (for example, a recombinant strain) expressing the variant of the present invention or the polynucleotide of the present invention; or a strain (for example, a recombinant strain) exhibiting the activity of the variant of the present invention, but is not limited thereto.

The strain of the present invention may be a strain having an L-tryptophan producing ability.

The strain of the present invention may be a microorganism naturally having tautomerase pptA or L-tryptophan producing ability or a microorganism in which the variant of the present invention or a polynucleotide encoding the variant (or a vector comprising the polynucleotide) is introduced into a parent strain that does not have a tautomerase pptA or L-tryptophan producing ability and/or in which an L-tryptophan producing ability is conferred on the parent strain, but is not limited thereto.

For example, the strain of the present invention may be a cell or microorganism that is transformed with a vector comprising the polynucleotide of the present invention or a polynucleotide encoding the variant of the present invention and expresses the variant of the present invention. For the purposes of the present disclosure, the strain of the present disclosure may include all microorganisms that comprise the variant of the present invention and can produce L-tryptophan. For example, the strain of the present disclosure may be a recombinant strain in which a polynucleotide encoding the variant of the present invention is introduced into a natural wild-type microorganism or a microorganism producing L-tryptophan to thus express a tautomerase pptA variant and having an increased L-tryptophan producing ability. The recombinant strain having an increased L-amino acid producing ability may be a microorganism having an increased L-tryptophan producing ability as compared to a natural wild-type microorganism or tautomerase pptA unmodified microorganism (namely, a microorganism expressing wild-type tautomerase pptA (SEQ ID NO: 3) or a microorganism that does not express modified (SEQ ID NO: 1) protein), but is not limited thereto. For example, the strain having an increased L-tryptophan producing ability of the present invention may be a microorganism having an increased L-tryptophan producing ability as compared to *Escherichia coli* which comprises a polypeptide of SEQ ID NO: 3 or a polynucleotide encoding the polypeptide, but is not limited thereto. As an example, the tautomerase pptA unmodified microorganism that is a target strain for comparison of the increase in the L-tryptophan producing ability may be an CJ600 strain (KCCM10812P, KR 10-0792095), but is not limited thereto.

For example, the recombinant strain having increased producing ability may have an L-tryptophan producing ability increased by about 1% or more, about 2.5% or more, about 5% or more, about 6% or more, about 7% or more, about 10% or more, about 12% or more, about 15% or more, about 17% or more, about 19% or more, or about 20% or more (the upper limit is not particularly limited and may be, for example, about 200% or less, about 150% or less, about 100% or less, about 50% or less, about 45% or less, about 40% or less, about 35% or less, about 30% or less, about 25% or less, about 22% or less, or about 21% or less) as compared to the L-tryptophan producing ability of the parent strain before being varied or an unmodified microorganism, but the increased amount is not limited thereto as long as the producing ability has an increased amount of a + value as compared to the producing ability of the parent strain before being varied or an unmodified microorganism. In another example, the recombinant strain having increased producing ability may have an L-tryptophan producing ability increased by about 1.01 times or more, about 1.02 times or more, about 1.05 times or more, about 1.07 times or more, about 1.1 times or more, about 1.12 times or more, about 1.15 times or more, about 1.17 times or more, about 1.19 times or more, or about 1.2 times or more (the upper limit is not particularly limited and may be, for example, about 10 times or less, about 5 times or less, about 3 times or less, about 2 times or less, about 1.5 times or less, or about 1.3 times or less) as compared to the L-tryptophan producing ability of the parent strain before being varied or an unmodified microorganism, but the rate of increase is not limited thereto. The term "about" is a range including all of ±0.5, ±0.4, ±0.3, ±0.2, ±0.1, and the like, includes all values in a range equal to or similar to the value following the term "about", but is not limited thereto.

In the present disclosure, the "unmodified microorganism" does not exclude strains comprising mutation that may occur naturally in microorganisms, and may be a wild-type strain or a natural strain itself or may be a strain before the trait is changed by genetic variation due to natural or artificial factors. For example, the unmodified microorganism may be a strain into which the tautomerase pptA variant described in the present specification is not introduced or has not yet been introduced. The term "unmodified microorganism" may be used interchangeably with "strain before being modified", "microorganism before being modified", "unvaried strain", "unmodified strain", "unvaried microorganism", or "reference microorganism".

In another example of the present disclosure, the microorganism of the present disclosure may be *Escherichia coli.*

In the present disclosure, the "weakening" of a polypeptide includes both cases where the activity is decreased or as compared to the endogenous activity or absent. The weakening may be used interchangeably with terms such as inactivation, deficiency, down-regulation, decrease, reduce, and attenuation.

The weakening may also include a case where the activity of the polypeptide itself is decreased or eliminated as compared to the activity of the polypeptide originally possessed by the microorganism by variation of the polynucleotide encoding the polypeptide, and the like, a case where the overall polypeptide activity level and/or concentration (expression level) in the cell is lower as compared to that of the natural strain by inhibition of the expression of the gene of the polynucleotide encoding the polypeptide or inhibition of translation into the polypeptide, a case where the polynucleotide is not expressed at all, and/or a case where the polypeptide activity is not exhibited even when the polynucleotide is expressed. The "endogenous activity" means the activity of a specific polypeptide originally possessed by the parent strain before the trait is changed or a wild-type or unmodified microorganism when the trait is changed by genetic variation due to natural or artificial factors. The endogenous activity may be used interchangeably with "activity before modification". The fact that the activity of a polypeptide is "inactivated, deficient, decreased, down-regulated, reduced, or attenuated" as compared to the endogenous activity means that the activity of a polypeptide is lowered as compared to the activity of a specific polypeptide originally possessed by the parent strain before the trait is changed or a wild-type or unmodified microorganism.

Such weakening of the activity of a polypeptide may be performed by any method known in the art, but the method is not limited thereto, and the weakening may be achieved by applying various methods well known in the art (for example, Nakashima N. et al., Bacterial cellular engineering by genome editing and gene silencing. Int J Mol Sci. 2014; 15(2):2773-2793, Sambrook et al., Molecular Cloning 2012, and the like).

Specifically, the weakening of the polypeptide activity in the present disclosure may be:
1) deletion of the entirety or a part of a gene encoding a polypeptide;
2) modification of an expression regulatory region (or expression control sequence) to decrease the expression of a gene encoding a polypeptide;
3) modification of an amino acid sequence constituting the polypeptide to eliminate or weaken the activity of the polypeptide (for example, deletion/substitution/addition of one or more amino acids in an amino acid sequence);
4) modification of a gene sequence encoding a polypeptide to eliminate or weaken the activity of the polypeptide (for example, deletion/substitution/addition of one or more nucleic acid bases in a nucleic acid base sequence of the polypeptide gene to encode a polypeptide that has been modified to eliminate or weaken the activity of the polypeptide);
5) modification of a start codon of a gene transcript encoding a polypeptide or a base sequence encoding a 5'-UTR region;
6) introduction of an antisense oligonucleotide (for example, antisense RNA) that complementarily binds to the transcript of the gene encoding a polypeptide;
7) addition of a sequence complementary to a Shine-Dalgarno sequence in front of the Shine-Dalgarno sequence of a gene encoding a polypeptide in order to form a secondary structure to which ribosome cannot be attached;
8) addition of a promoter to be transcribed in the opposite direction to the 3' end of the open reading frame (ORF) of a gene sequence encoding a polypeptide (reverse transcription engineering, RTE); or
9) a combination of two or more selected from 1) to 8), but is not particularly limited thereto.

For example:
1) The deletion of a part or the entirety of the gene encoding a polypeptide may be removal of the entire polynucleotide encoding the endogenous polypeptide of interest in the chromosome, replacement with a polynucleotide in which some nucleotides are deleted or replacement with a marker gene.
2) The modification of the expression regulatory region (or expression control sequence) may be deletion, insertion, non-conservative or conservative substitution, or occurrence of variation in the expression regulatory region (or expression control sequence) due to a combination thereof or replacement with a sequence exhibiting weaker activity. The expression regulatory region contains a promoter, an operator sequence, a sequence encoding a ribosome binding site, and a sequence controlling the termination of transcription and translation, but is not limited thereto.
5) The modification of a start codon of a gene transcript encoding a polypeptide or a base sequence encoding a 5'-UTR region may be, for example, substitution with a base sequence encoding another start codon having a lower polypeptide expression rate as compared to the endogenous start codon, but is not limited thereto.
3) and 4) The modification of an amino acid sequence or polynucleotide sequence may be deletion, insertion, or non-conservative or conservative substitution of an amino acid sequence of the polypeptide or a polynucleotide sequence encoding the polypeptide or occurrence of variation in the sequence due to a combination thereof or replacement with an amino acid sequence or polynucleotide sequence modified to exhibit weaker activity or an amino acid sequence or polynucleotide sequence modified to be inactive so that the activity of the polypeptide is weakened, but is not limited thereto. For example, the expression of a gene may be inhibited or weakened by introducing variation into the polynucleotide sequence and forming a stop codon, but the modification is not limited thereto.
6) For the introduction of an antisense oligonucleotide (for example, antisense RNA) that complementarily binds to the transcript of the gene encoding a polypeptide, reference may be made to documents, for example, Weintraub, H. et al., Antisense-RNA as a molecular tool for genetic analysis, Reviews-Trends in Genetics, Vol. 1(1) 1986.
7) The addition of a sequence complementary to a Shine-Dalgarno sequence in front of the Shine-Dalgarno sequence of a gene encoding a polypeptide in order to form a secondary structure to which ribosome cannot be attached may be to make mRNA translation impossible or to slow down the mRNA translation rate.
8) The addition of a promoter to be transcribed in the opposite direction to the 3' end of the open reading frame (ORF) of a gene sequence encoding a polypeptide (reverse transcription engineering, RTE) may be to weaken the activity by making an antisense nucleotide complementary to the transcript of the gene encoding a polypeptide.

In the present disclosure, the term "enhancement" of polypeptide activity means that the activity of a polypeptide is increased as compared to the endogenous activity. The enhancement may be used interchangeably with terms such as activation, up-regulation, overexpression, and increase. Here, activation, enhancement, up-regulation, overexpression, and increase may include both exhibiting activity that was not originally possessed and exhibiting improved activity as compared to the endogenous activity or activity before modification. The "endogenous activity" means the activity of a specific polypeptide originally possessed by the parent strain before the trait is changed or an unmodified microorganism when the trait is changed by genetic variation due to natural or artificial factors. This may be used interchangeably with "activity before modification". The fact that the activity of a polypeptide is "enhanced", "up-regulated", "overexpressed", or "increased" as compared to the endogenous activity means that the activity of a polypeptide is improved as compared to the activity and/or concentration (expression level) of a specific polypeptide originally possessed by the parent strain before the trait is changed or an unmodified microorganism.

The enhancement may be achieved through the introduction of a foreign polypeptide or the enhancement of endogenous activity and/or concentration (expression level) of the polypeptide. The enhancement of the activity of a polypeptide may be confirmed by an increase in the degree of activity and the expression level of the polypeptide or in the amount of the product produced from the polypeptide.

For the enhancement of the activity of a polypeptide, various methods well known in the art may be applied, and the method is not limited as long as the activity of the polypeptide of interest can be enhanced as compared to that of the microorganism before being modified. Specifically, genetic engineering and/or protein engineering well known to those skilled in the art, which are routine methods of molecular biology, may be used, but the method is not limited thereto (for example, Sitnicka et al., Functional Analysis of Genes. Advances in Cell Biology. 2010, Vol. 2. 1-16; Sambrook et al., Molecular Cloning 2012; and the like).

Specifically, the enhancement of the activity of a polypeptide of the present disclosure may be:
1) increase in the intracellular copy number of a polynucleotide encoding a polypeptide;
2) replacement of a gene expression regulatory region on a chromosome encoding a polypeptide with a sequence exhibiting strong activity;
3) modification of a start codon of a gene transcript encoding a polypeptide or a base sequence encoding a 5'-UTR region;
4) modification of an amino acid sequence of a polypeptide to enhance the activity of the polypeptide;
5) modification of a polynucleotide sequence encoding a polypeptide to enhance the activity of the polypeptide (for example, modification of a polynucleotide sequence of the polypeptide gene to encode a polypeptide that has been modified to enhance the activity of the polypeptide);
6) introduction of a foreign polypeptide exhibiting the activity of a polypeptide or a foreign polynucleotide encoding the polypeptide;
7) codon optimization of a polynucleotide encoding a polypeptide;
8) analysis of the tertiary structure of a polypeptide to select and modification or chemical modification of the exposed site; or
9) a combination of two or more selected from 1) to 8), but is not particularly limited thereto.

More specifically:
1) The increase in the intracellular copy number of a polynucleotide encoding a polypeptide may be achieved by the introduction into a host cell of a vector which can be replicated and function independently of the host and to which a polynucleotide encoding the polypeptide is operably linked. Alternatively, the increase may be achieved by the introduction of one copy or two or more copies of a polynucleotide encoding the polypeptide into a chromosome of a host cell. The introduction into the chromosome may be performed by introducing a vector capable of inserting the polynucleotide into a chromosome of a host cell into the host cell, but is not limited thereto. The vector is as described above.
2) The replacement of a gene expression regulatory region (or expression control sequence) on a chromosome encoding a polypeptide with a sequence exhibiting strong activity may be, for example, deletion, insertion, non-conservative or conservative substitution, or occurrence of variation in a sequence due to a combination thereof or replacement with a sequence exhibiting stronger activity so that the activity of the expression regulatory region is further enhanced. The expression regulatory region is not particularly limited thereto, but may contain a promoter, an operator sequence, a sequence encoding a ribosome binding site, a sequence controlling the termination of transcription and translation, and the like. For example, the replacement may be to replace the original promoter with a strong promoter, but is not limited thereto.
   Examples of known strong promoters include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, and yccA promoter, but are not limited thereto.
3) The modification of a start codon of a gene transcript encoding a polypeptide or a base sequence encoding a 5'-UTR region may be, for example, substitution with a base sequence encoding another start codon having a higher polypeptide expression rate as compared to an endogenous start codon, but is not limited thereto.
4) and 5) The modification of an amino acid sequence or polynucleotide sequence may be deletion, insertion, non-conservative or conservative substitution of an amino acid sequence of the polypeptide or a polynucleotide sequence encoding the polypeptide or occurrence of variation in the sequence due to a combination thereof or replacement with an amino acid sequence or polynucleotide sequence modified to exhibit stronger activity or an amino acid sequence or polynucleotide sequence modified to be more active so that the activity of the polypeptide is enhanced, but is not limited thereto. The replacement may be specifically performed by inserting a polynucleotide into a chromosome by homologous recombination, but is not limited thereto. The vector used here may further contain a selection marker for the confirmation of chromosome insertion. The selection marker is as described above.
6) The introduction of a foreign polypeptide exhibiting the activity of a polypeptide may be the introduction of a foreign polynucleotide encoding a polypeptide exhibiting activity the same as or similar to that of the polypeptide into a host cell. The foreign polynucleotide is not limited in its origin or sequence as long as it exhibits activity the same as or similar to that of the polypeptide. The introduction may be performed by appropriately selecting a known transformation method by those skilled in the art. As the introduced polynucleotide is expressed in a host cell, a polypeptide may be produced, and the activity thereof may be increased.
7) The codon optimization of a polynucleotide encoding a polypeptide may be codon optimization of an endogenous polynucleotide so as to increase transcription or translation in a host cell or codon optimization of a foreign polynucleotide so as to perform optimized transcription and translation in a host cell.
8) The analysis of the tertiary structure of a polypeptide to select and modification or chemical modification of the exposed site may be, for example, to determine a template protein candidate according to the degree of similarity of the sequence by comparing the sequence information of a polypeptide to be analyzed with a database storing the sequence information of known proteins, to confirm the structure based on this, and to select and modify or chemically modify the exposed portion to be modified or chemically modified.

Such enhancement of the polypeptide activity may be an increase in the activity or concentration expression level of the corresponding polypeptide based on the activity or concentration of the polypeptide expressed in a wild-type microbial strain or a microbial strain before being modified or an increase in the amount of a product produced from the polypeptide, but is not limited thereto.

In the microorganism of the present disclosure, partial or entire modification of a polynucleotide may be induced by (a) homologous recombination using a vector for chromosome insertion in the microorganism or genome editing using engineered nuclease (*e.g*., CRISPR-Cas9) and/or (b) treatment with light such as ultraviolet rays and radiation and/or chemicals, but is not limited thereto. A method for modifying a part or the entirety of the gene may include a method using DNA recombination technology. For example, by introducing a nucleotide sequence or vector containing a nucleotide sequence homologous to the gene of interest into the microorganism to cause homologous recombination, a part or the entirety of the gene may be deleted. The introduced nucleotide sequence or vector may contain a dominant selection marker, but is not limited thereto.

In the microorganism of the present disclosure, the variant, polynucleotide, L-tryptophan, and the like are as described in the other aspects.

Still another aspect of the present invention provides a method for producing L-tryptophan, which includes culturing *an Escherichia coli* strain comprising the variant of the present invention or the polynucleotide of the present invention in a medium.

The method for producing L-tryptophan of the present invention may include culturing *an Escherichia coli* strain comprising the variant of the present invention or the polynucleotide of the present invention or the vector of the present disclosure in a medium.

In the present invention, the term "culture" means growing the *Escherichia coli* strain of the present invention under appropriately controlled environmental conditions. The culture process of the present invention may be performed according to suitable medium and culture conditions known in the art. Such a culture process may be easily adjusted and used by those skilled in the art according to the selected strain. Specifically, the culture may be a batch type, continuous type, and/or fed-batch type, but is not limited thereto.

In the present disclosure, the term "medium" means a mixed substance containing nutrients required to culture the *Escherichia coli* strain of the present disclosure as a main component, and the medium supplies nutrients, growth factors, and the like including water that are indispensable for survival and development. Specifically, as the medium and other culture conditions used for culture of the *Escherichia coli* strain of the present invention, any one may be used without particular limitation as long as it is a medium used for common culture of microorganisms. The *Escherichia coli* strain of the present invention may be cultured in a common medium containing proper carbon sources, nitrogen sources, phosphorus sources, inorganic compounds, amino acids and/or vitamins, and the like while controlling the temperature, pH, and the like under aerobic conditions.

Specifically, the culture medium for the *Escherichia coli* strain may be found in the document "Manual of Methods for General Bacteriology" by the American Society for Bacteriology (Washington, D.C., USA, 1981).

In the present disclosure, the carbon sources include carbohydrates such as glucose, saccharose, lactose, fructose, sucrose, and maltose; sugar alcohols such as mannitol and sorbitol, organic acids such as pyruvic acid, lactic acid, and citric acid; amino acids such as glutamic acid, methionine, and lysine; and the like. Natural organic nutrients such as starch hydrolysate, molasses, blackstrap molasses, rice bran, cassava, sugarcane residue, and corn steep liquor may be used. Specifically, carbohydrates such as glucose and sterilized pretreated molasses (namely, molasses converted to reducing sugar) may be used, and appropriate amounts of other carbon sources may be used in various manners without limitation. These carbon sources may be used singly or in a combination of two or more, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate; and organic nitrogen sources such as amino acids such as glutamic acid, methionine, and glutamine, peptone, NZ-amine, meat extract, yeast extract, malt extract, corn steep liquor, casein hydrolysate, fish or decomposition products thereof, and skim soybean cake or decomposition products thereof may be used. These nitrogen sources may be used singly or in a combination of two or more, but are not limited thereto.

The phosphorus sources may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, and the like may be used. In addition to these, amino acids, vitamins and/or suitable precursors, and the like may be contained. These components or precursors may be added to the medium batchwise or continuously, but the manner of addition is not limited thereto.

During the culture of the *Escherichia coli* strain of the present invention, the pH of the medium may be adjusted by adding compounds such as ammonium hydroxide, potassium hydroxide, ammonia, phosphoric acid, and sulfuric acid to the medium in a proper manner. During the culture, foaming may be suppressed by using an antifoaming agent such as fatty acid polyglycol ester. Oxygen or oxygen-containing gas may be injected into the medium in order to maintain the aerobic state of the medium, or gas may not be injected or nitrogen, hydrogen, or carbon dioxide gas may be injected in order to maintain the anaerobic and microaerobic states, but the method for maintaining the state is not limited thereto.

In the culture of the present invention, the culture temperature may be maintained at 20°C to 45°C, specifically 25°C to 40°C, and the strain may be cultured for about 10 to 160 hours, but the culture conditions are not limited thereto.

The L-tryptophan produced through the culture of the present invention may be secreted into the medium or may remain in the cells.

The method for producing L-tryptophan of the present invention may further comprise a step of preparing the *Escherichia coli* strain of the present invention, a step of preparing a medium for culture of the strain, or a combination of these (in any order), for example, prior to the culture step.

The method for producing L-tryptophan of the present invention may further include a step of recovering the L-tryptophan from the medium according to the culture (the medium subjected to the culture) or from the *Escherichia coli* strain. The recovery step may be further included after the culture step.

The recovery may be to collect the L-tryptophan by way of a suitable method known in the art according to the method for culturing the microorganism of the present invention, for example, a batch, continuous, or fed-batch culture method. For example, centrifugation, filtration, treatment with a crystallized protein precipitant (salting out), extraction, ultrasonic disintegration, ultrafiltration, dialysis, various forms of chromatography such as molecular-sieve chromatography (gel filtration), adsorption chromatography, ion-exchange chromatography, and affinity chromatography, HPLC, or a combination thereof may be used. The L-tryptophan may be recovered from the medium or microorganism by way of a suitable method known in the art.

The method for producing L-tryptophan of the present invention may further include a purification step. The purification may be performed by way of a suitable method known in the art. In an example, when the method for producing L-tryptophan of the present invention includes both the recovery step and the purification step, the recovery step and the purification step may be performed continuously or discontinuously regardless of the order, or may be performed simultaneously or by being combined into one step, but the manner to perform the steps is not limited thereto.

In the method of the present invention, the variant, polynucleotide, vector, strain, and the like are as described in the other aspects.

Still another aspect of the present disclosure is to provide a composition for L-tryptophan production, which comprises *an Escherichia coli* strain comprising the variant of the present invention, a polynucleotide encoding the variant, a vector comprising the polynucleotide, or the polynucleotide of the present invention; a medium in which this *Escherichia coli* strain has been cultured; or a combination of two or more of these.

The composition of the present disclosure may further comprise arbitrary suitable excipients to be commonly used in compositions for L-tryptophan production. Such excipients may be, for example, a preservative, a wetting agent, a dispersing agent, a suspending agent, a buffering agent, a stabilizer, or an isotonic agent, but are not limited thereto.

In the composition of the present disclosure, the variant, polynucleotide, vector, strain, medium, L-tryptophan, and the like are as described in the other aspects.

### [Mode for Invention]

Hereinafter, the present invention will be described in more detail by way of Examples. However, the following Examples are only preferred embodiments for illustrating the present invention, and thus are not intended to limit the scope of the present invention thereto. Meanwhile, technical matters not described in the present specification can be sufficiently understood and easily implemented by those skilled in the technical field of the present invention or similar technical fields.

### Example 1. Construction of strain for expressing for tautomerase pptA variant

In order to construct an *E. coli* strain with a pptA variant (R31H; SEQ ID NO: 1) phenotype, fragments for the upstream region and the downstream region of the *E. coli* pptA R31H variant were each obtained by performing PCR using the *E*. *coli* W3110 gDNA as a template along with a primer pair of SEQ ID NOS: 5 and 6 and a primer pair of SEQ ID NOS: 7 and 8. SoIg^{™} Pfu-X DNA polymerase was used as a polymerase, and PCR amplification was performed as follows: denaturation at 95°C for 2 minutes; 27 cycles of denaturation at 95°C for 30 seconds, annealing at 55°C for 1 minute, and polymerization at 72°C for 1 minute; and polymerization at 72°C for 5 minutes. To induce homologous recombination on the chromosome, a recombinant plasmid was obtained by cloning each of the amplified upstream region and the downstream region of the pptA R31Hvariant and the pSG76-C plasmid, which is a vector for chromosomal transformation digested with Smal restriction enzyme (Journal Of Bacteriology, July 1997, p. 4426-4428) using the Gibson Assembly, and named pSG76-C-pptA(R31H). The cloning was performed by mixing the Gibson assembly reagent and each of the gene fragments at a calculated mole number followed by storing at 50°C for one hour. CJ600 strain (KR 10-0792095) was transformed with the pSG76-C-pptA(R31H) plasmid, cultured in LB-Cm medium (yeast extract 10 g/L, NaCl 5 g/L, tryptone 10 g/L, chlororamphenicol 25 µg/L), and then, colonies with chloramphenicol resistance were selected. The selected transformant was confirmed to be a strain in which the pSG76-C-pptA(R31H) plasmid was inserted into the *pptA* gene region in the chromosome using a primer pair of SEQ ID NOS: 9 and 10. The strain, which was first confirmed that into which the pptA(R31H)variant was inserted, was transformed with the pST76-AsceP (Nucleic Acids Research, Volume 27, Issue 22, 1 November 1999, Pages 4409-4415), and colonies which grew in the LB-Amp(Yeast extract 10g/L, NaCl 5g/L, Tryptone 10g/L, Ampicillin 100ug/L) solid medium at 30°C were selected. The selected colonies were picked in LB, LB-Amp, and LB-Cm solid medium, respectively, and cultured at 42°C for 16 hours. It was confirmed that the pST76-AsceP plasmid was cured in the colonies which grew on LB solid medium but did not grow on LB-Amp, LB-Cm solid medium. Finally, in the colonies where the pST76-AsceP plasmid was confirmed to be cured, the *pptA* gene was amplified using a pair of primers of SEQ ID NOS: 9 and 10, respectively, and a strain in which the *pptA* gene was replaced with pptA(R31H), was selected by sequencing. The thus-constructed strain was named CJ600_pptA_R31H.

### Example 2. Evaluation on L-tryptophan producing ability of microorganism expressing tautomerase pptA variant

The L-tryptophan producing abilities of each of the strains prepared in Example 1 and the parent strain (the control strain) were analyzed by evaluation of the flask fermentation titer of these strains.

Colonies of the control parent strain, CJ600, and the recombinant strain CJ600_pptA_R31 Hprepared in Example 1 were cultured overnight in LB solid medium using inoculation loop. The grown strains were each inoculated in a 250 mL corner-baffle flask containing 25 mL of the production medium, and cultured with shaking at 37°C at 200 rpm for 48 hours. After the completion of the culture, the L-tryptophan producing abilities of these colonies were measured by HPLC.

### <Production Medium (pH 7.0)>

glucose 60 g, yeast extract 2.5 g, ammonium sulfate [(NH₄)₂SO₄·7H₂O] 20 g, magnesium sulfate (MgSO₄) 1 g, potassium dihydrogen phosphate (KH₂PO₄) 2 g, sodium citrate (Na-citrate) 5 g , sodium chloride (NaCl) 1 g, tyrosine (L-tyrosine) 0.1 g, L-phenylalanine 0.15 g, calcium carbonate (CaCOs) 40 g (based on 1 liter of distilled water)

The experiment was repeated 3 times and the average values of the analysis results are shown in Table 1 below.

**[Table 1]**

| Comparison of L-Tryptophan producing ability | |
|---|---|
| Strain | L-Tryptophan concentration (g/L) |
| CJ600 | 6.5 |
| CJ600_pptA_R31H | 7.8 |

As presented in Table 1, the CJ600_pptA_R31Hstrain showed an increased L-tryptophan producing ability compared to the control group.

The CJ600_pptA_R31H strain was named CA04-4593, deposited with the Korea Culture Center of Microorganisms, a depository institution under the Budapest Treaty, on December 2, 2020, and given the accession number KCCM 12885P.

## Claims

1. A tautomerase pptA variant consisting of an amino acid sequence represented by SEQ ID NO: 1, wherein arginine, which is an amino acid corresponding to position 31 of SEQ ID NO: 3, is substituted with histidine.

2. A polynucleotide encoding the variant according to claim 1.

3. An *Escherichia coli* strain comprising the variant according to claim 1 or a polynucleotide encoding the variant.

4. The strain according to claim 3, which has increased L-tryptophan producing ability as compared to *Escherichia coli* comprising a polypeptide of SEQ ID NO: 3 or a polynucleotide encoding the polypeptide.

5. A method for producing L-tryptophan, the method comprising culturing an *Escherichia coli* strain comprising the variant according to claim 1 or a polynucleotide encoding the variant in a medium.

## Patentansprüche

1. Tautomerase-pptA-Variante, bestehend aus einer durch SEQ ID NO: 1 dargestellten Aminosäuresequenz, wobei Arginin, das eine Aminosäure entsprechend Position 31 von SEQ ID NO: 3 ist, durch Histidin ersetzt ist.

2. Polynucleotid, das für eine Variante nach Anspruch 1 kodiert.

3. Escherichia-coli-Stamm, umfassend eine Variante nach Anspruch 1 oder ein für die Variante kodierendes Polynucleotid.

4. Stamm nach Anspruch 3, der eine erhöhte L-Tryptophan-Produktionsfähigkeit verglichen mit Escherichia coli, umfassend ein Polypeptid von SEQ ID NO: 3 oder ein für das Polypeptid kodierendes Polynucleotid, aufweist.

5. Verfahren zur Produktion von L-Tryptophan, wobei das Verfahren das Kultivieren eines Escherichia-coli-Stamms, umfassend eine Variante nach Anspruch 1 oder ein für die Variante kodierendes Polynucleotid, in einem Medium umfasst.

## Revendications

1. Variant de tautomérase pptA constitué d'une séquence d'acides aminés représentée par SEQ ID NO : 1, dans lequel l'arginine, qui est un acide aminé correspondant à la position 31 de SEQ ID NO : 3, est substitué par de l'histidine.

2. Polynucléotide codant pour le variant selon la revendication 1.

3. Souche d'*Escherichia coli* comprenant le variant selon la revendication 1 ou un polynucléotide codant pour le variant.

4. Souche selon la revendication 3, qui présente une capacité de production de L-tryptophane accrue par rapport à *Escherichia coli,* comprenant un polypeptide de SEQ ID NO : 3 ou un polynucléotide codant pour le polypeptide.

5. Procédé de production de L-tryptophane, le procédé comprenant la mise en culture d'une souche d'*Escherichia coli* comprenant le variant selon la revendication 1 ou un polynucléotide codant pour le variant dans un milieu.
